# EUROPÄISCHE PATENTSCHRIFT

(11) **EP 0 843 990 B1**
(45) Veröffentlichungstag und Bekanntmachung des Hinweises auf die Patenterteilung: **30.07.2003**
(21) Anmeldenummer: 96203188.6
(22) Anmeldetag: 15.11.1996
(51) Int. Cl.: A61F 2/06, A61M 25/10

(54) **Ballonkatheter und Vorrichtung zum Setzen eines Stents**
Balloon catheter and delivery device for a stent
Cathéter à ballon et système d'application d'une endoprothèse

(43) Veröffentlichungstag der Anmeldung: 27.05.1998
(73) Patentinhaber: Schneider (Europe) GmbH, 8180 Bülach (CH)
(72) Erfinder: Berger, Erwin, 8585 Mattwil (CH); Amann, Rainer, 79771 Klettgau/Riedern a.S. (DE)
(74) Vertreter: Kiliaridis, Constantin

(56) Entgegenhaltungen:
- EP-A- 0 727 194

## Beschreibung

Die Erfindung bezieht sich auf einen Ballonkatheter mit einem rohrförmigen Katheterschaft, der ein proximales und ein distales Ende aufweist, einem länglichen Dilatationsballon, der am Katheterschaft in der Nähe des distalen Endes angeordnet ist, und einer längs des Katheterschaftes verschiebbaren, durchmesserstabilen Abdeckhülse, die einen den Katheterschaft umfassenden ersten Abschnitt mit einem ersten Innendurchmesser und einen über den deflatierten Dilatationsballon schiebbaren zweiten Abschnitt mit einem zweiten Innendurchmesser aufweist, der grösser als der erste Innendurchmesser ist.

Die Erfindung bezieht sich ausserdem auf eine Vorrichtung zum Setzen eines ballonexpandierbaren Stents, die einen Ballonkatheter der eingangs genannten Art aufweist.

In der Ballonkathetertechnologie ist die Verwendung von Ballonkathetern mit fester Ballonlänge bekannt. Viele medizinische Anwendungen erfordern den Einsatz mehrerer Ballonkatheter mit unterschiedlichen Ballonlängen. Verlangt ein Eingriff beispielsweise zwei unterschiedlich lange Ballone, so ist es notwendig, den Ballonkatheter zu wechseln oder mit einem Ballon mehrmals aufeinanderfolgend zu arbeiten. Ein Ballonkatheterwechsel ist aber sehr kostspielig und ein sequentielles Arbeiten zeitaufwendig. Darüber hinaus wird das Verletzungsrisiko für den Patienten erhöht oder es kann zu unzureichenden Dilatationen führen.

Ein Ballonkatheter ist ausserdem ein gebräuchliches Instrument für den Transport und das Aufweiten einer ballonexpandierbaren Endoprothese, genannt Stent, die zur Aufrechterhaltung der Durchgängigkeit eines Gefässes gesetzt wird. Die Ballonlänge ist dabei in Abhängigkeit der Länge des Stents auszuwählen, um eine fehlerhafte Expansion des Stents und eine damit verbundene Beschädigung des Gefässes zu vermeiden. Dies führt zwangsläufig zum aufwendigen Bedarf einer grösseren Auswahl an Ballonkathetern mit unterschiedlichen Ballonlängen, um Stents vorschriftsmässig und sicher anwenden zu können.

Die EP 0 727 194 A1 zeigt dazu einen Ballonkatheter der eingangs erwähnten Art, bei dem eine durchmesserstabile Abdeckhülse zwei Positionen einnehmen kann. In ihrer proximalen Endposition gibt die Abdeckhülse die volle Ballonlänge zur Expansion frei, während sie in ihrer distalen Endposition einen proximalen Ballonabschnitt abgedeckt und von einer Expansion bis zum Nenndurchmesser des Ballons abhält. Dadurch kann bei einem solchen Ballonkatheter zwischen zwei verschiedenen Ballonlängen ausgewählt werden. Die distale Endposition wird reibschlüssig durch Schieben der Abdeckhülse auf eine Verdickungsstelle am Katheterschaft gesichert. Die Abdeckhülse weist auch ein verjüngtes distales Ende ohne Verstärkungsmittel auf, das jedoch im Gebrauch durch den unter Druck gesetzten Dilatationsballon aufgeweitet wird. Der vollexpandierte Teil des Dilatationsballons übt dann eine proximal gerichtete Kraft gegen die Abdeckhülse aus, wodurch diese zu migrieren beginnen kann. Zwischenstellungen der Abdeckhülse und damit Zwischenwerte der Ballonlänge sind bei diesem Katheter nicht wählbar. In diesem Fall wäre die Abdeckhülse nicht in ihrer Lage gesichert und würde unter dem Dilatationsdruck migrieren.

Der Erfindung liegt nun die Aufgabe zugrunde, einen Ballonkatheter der eingangs genannten Art anzugeben, bei dem die Ballonlänge kontinuierlich eingestellt werden kann und eine eingestellte Ballonlänge beim Inflatieren des Dilatationsballons sicher haltbar ist. Darüberhinaus soll eine Vorrichtung zum Setzten ballonexpandierbarer Stents unterschiedlicher Längen geschaffen werden, die vielseitig eingesetzbar sowie einfach und sicher in der Handhabung ist.

Die Aufgabe wird gemäss der Erfindung gelöst durch einen Ballonkatheter der eingangs genannten Art mit den Merkmalen des kennzeichnenden Teils des Patentanspruchs 1 und durch eine Vorrichtung zum Setzten eines ballonexpandierbaren Stents nach Patentanspruch 15.

Wenn die Abdeckhülse am distalen Ende des zweiten Abschnitts einen durchmesserstabilen dritten Abschnitt mit einem dritten Innendurchmesser aufweist, der zwischen dem ersten Innendurchmesser und dem zweiten Innendurchmesser liegt, wird dieser dritte Abschnitt zwischen dem vom zweiten Abschnitt abgedeckten, teilexpandierbaren Ballonabschnitt und dem freien, vollexpandierbaren Ballonabschnitt beim Inflatieren des Dilatationsballons stabil eingeschlossen. Die Abdeckhülse wird in diesem Einschluss durch den Dilatationsdruck in ihrer axialen Position gehalten, so dass ein stufenloses Einstellen der Ballonlänge ohne zusätzliche reibschlüssige Sicherungsmittel erreicht ist. Im praktischen Einsatz kann ein erfindungsgemässer Ballonkatheter, sei es für die Dilatation von Stenosen oder zur Stentimplantation, sowohl als Over-the-wire-Katheter als auch als MONORAIL-Katheter (Trademark) ausgeführt sein. In beiden Fällen ist es prinzipiell möglich, die Abdeckhülse entweder von proximal oder von distal auf den deflatierten Dilatationsballon zu schieben und so die frei expandierbare Ballonlänge kontinuierlich einzustellen.

In einer bevorzugten Ausführungsform der Erfindung ist die Abdeckhülse von proximal auf den deflatierten Dilatationsballon schiebbar, wobei der erste Abschnitt proximal und der dritte Abschnitt distal vom zweiten Abschnitt der Abdeckhülse angeordnet ist. Dadurch muss die Abdeckhülse beim Einführen des Ballonkatheters in ein Blutgefäss nicht durch die Verengungsstelle hindurchgeführt werden, sondern sie nimmt nach Positionierung des Dilatationsballons eine Stellung proximal von der Stenose ein.

In einer vorteilhaften Ausgestaltung der Erfindung sind der zweite Abschnitt der Abdeckhülse und einer der anderen Abschnitte einstückig ausgebildet und der andere äussere Abschnitt in den zweiten Abschnitt eingesetzt, was fertigungstechnische Vorteile bietet.

Vorzugsweise sind einstückig ausgebildete Abschnitte der Abdeckhülse mit einer durchmesserstabilen, flexiblen Armierung ausgeführt, um einerseits den hohen Inflationsdrücken vom Dilatationsballon standhalten zu können und andererseits sich engen Gefässwindungen beim Vorschieben des Ballonkatheters in einem Gefässsystem anpassen zu können.

In einer weiteren bevorzugten Ausführungsform der Erfindung wird der eingesetzte Abschnitt der Abdeckhülse durch ein den Katheterschaft eng umfassendes Spannelement gebildet. Der durch die Spannkraft erzeugte Reibschluss zwischen Spannelement und Katheterschaft fixiert eine voreingestellte axiale Position der Abdeckhülse auf dem Katheterschaft. Dadurch wird vermieden, dass sich beim Einführen des Ballonkatheters in den Körper eines Patienten eine eingestellte Ballonlänge verstellen kann. Das Spannelement ist vorzugsweise eine längsgeschlitzte Spannhülse, da eine solche besonders einfach herstellbar ist. Durch den Schlitz ist ein fester Sitz der Spannhülse auch bei kleinen Fertigungstoleranzen gewährleistet.

In einer anderen vorteilhaften Ausgestaltung der Erfindung weist die Abdeckhülse einen die Armierung und das Spannelement überziehenden Hüllschlauch auf. Dieser verleiht der Abdeckhülse eine glatte, atraumatische Aussenfläche, wodurch die guten Führungseigenschaften des Ballonkatheters erhalten bleiben und das Verletzungsrisiko für den Patienten reduziert wird.

Der Katheterschaft weist ein proximales und ein distales Begrenzungselement auf, zwischen welchen die Abdeckhülse längs des Katheterschaftes verschiebbar ist. Dies sichert die Abdeckhülse gegen ein Abrutschen vom Katheterschaft. Das proximale Begrenzungselement ist vorzugsweise eine Schulter am Katheterschaft und das distale Begrenzungselement wird bevorzugt durch die proximale Befestigungsstelle des Dilatationsballons am Katheterschaft oder durch den gefalteten, deflatierten Dilatationsballon selbst gebildet. In letzterem Fall brauchen für das distale Begrenzungselement keine besonderen zusätzlichen Massnahmen getroffen werden. Die Begrenzungselemente stellen Vergrösserungen des Schaftdurchmessers dar, über die die Abdeckhülse aufgrund des den Katheterschaft eng umschliessenden Spannelements jeweils nicht hinwegschiebbar ist.

Die Armierung kann aus einem schraubenlinienförmig gewickelten Drahtmaterial bestehen oder als rohrförmiges Drahtgeflecht ausgebildet sein. Beide Ausführungen besitzen eine hohe Flexibilität und sind druckbeständig hinsichtlich der Durchmesserstabilität. Besteht die Armierung aus einem röntgendichten Material, so kann kann die Abdeckhülse während des Eingriffs auf einem Röntgenbildschirm sichtbar gemacht werden.

Mit dem oben beschriebenen Ballonkatheter ist auch das wesentliche Element einer Vorrichtung zum Setzen ballonexpandierbarer Stents unterschiedlicher Längen geschaffen. Der Vorteil einer erfindungsgemässen Vorrichtung besteht insbesondere darin, dass sie beispielsweise zunächst zum Vordilatieren einer Stenose mit einer ersten Ballonlänge und anschliessend zum Setzen eines Stents mit einer zweiten Ballonlänge verwendet werden kann, indem die Vorrichtung nach der Aufweitung der Stenose entlang eines Führungsdrahtes aus dem Gefäss gezogen wird, dann mittels der Abdeckhülse die neue Ballonlänge eingestellt wird und der zu implantierende Stent in seinem unexpandierten Zustand auf den frei expandierbaren Ballonabschnitt montiert wird. Anschliessend wird der Ballonkatheter wieder bis zur vordilatierten Gefässstelle geführt, um durch erneutes Inflatieren des Dilatationsballon den Stent in seinen expandierten Zustand zu überführen. Falls erforderlich, kann mit demselben Ballonkatheter und eventuell mit einer dritten Ballonlänge noch eine Nachdilatation erfolgen.

Weitere Vorteile eines erfindungsgemässen Ballonkatheters und einer erfindungsgemässen Vorrichtung zum Setzen eines ballonexpandierbaren Stents ergeben sich aus einem bevorzugten Ausführungsbeispiel, das im folgenden anhand der Zeichnungen näher beschrieben wird, in deren
- FIG. 1: eine Seitenansicht des Ballonkatheters, wobei die Abdeckhülse in ihre proximale Endposition geschoben ist, in
- FIG. 2: eine Seitenansicht der Vorrichtung zum Setzen eines ballonexpandierbaren Stents, wobei die Abdeckhülse des Ballonkatheters einen Teil des Dilatationsballons abdeckt, und in
- FIG. 3: eine Seitenansicht des Ballonkatheters, dessen Abdeckhülse in eine distale Position geschoben ist, dargestellt ist. In
- FIG. 4: ist die Abdeckhülse des Ballonkatheters durch einen axialen Längsschnitt und in
- FIG. 5: durch einen Querschnitt im Bereich der Spannhülse veranschaulicht.
- FIG. 6: zeigt die Anordnung aus FIG. 3 vergrössert, wobei die Abdeckhülse im Längsschnitt dargestellt ist.

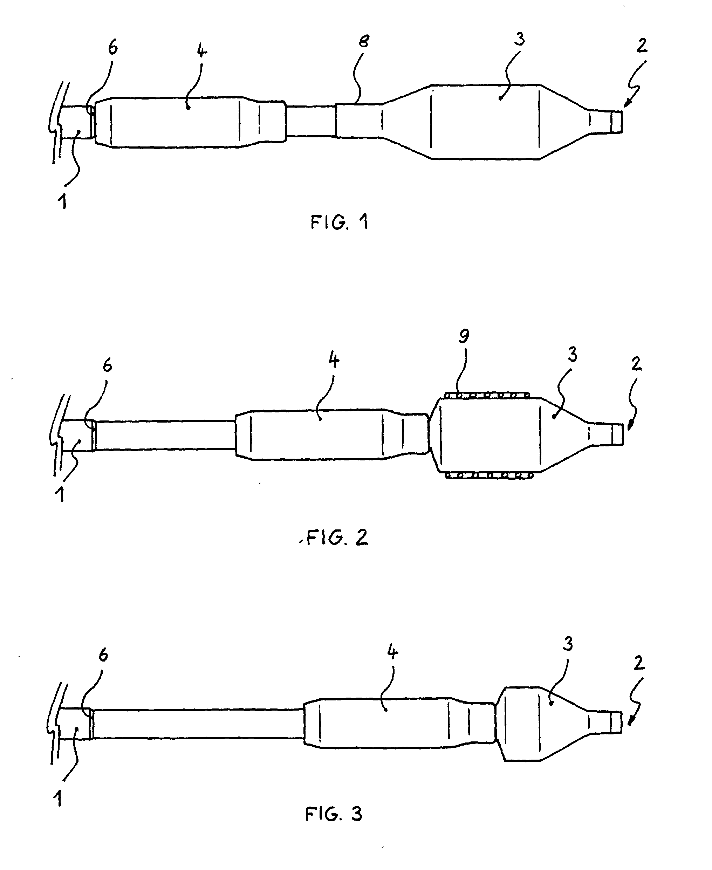

FIG. 1 bis FIG. 3 zeigt einen erfindungsgemässen Ballonkatheter mit einem rohrförmigen Katheterschaft 1, der ein proximales Ende (nicht dargestellt) und ein distales Ende 2 aufweist, an dem ein länglicher Dilatationsballon 3 angeordnet ist. Proximal vom Dilatationsballon 3 weist der Ballonkatheter eine durchmesserstabile Abdeckhülse 4 auf, die längs des Katheterschaftes 1 stufenlos zwischen einem proximalen Begrenzungselement , welches in diesem Ausführungsbeispiel eine Schulter 6 ist, und einem distalen Begrenzungselement verschiebbar ist, welches in diesem Fall durch die proximale Ballonbefestigungsstelle 8 des Dilatationsballons 3 am Katheterschaft 1 gebildet wird. Das distale Begrenzungselement 7 kann aber auch der gefaltete, deflatierte Dilatationsballon 3 selbst sein. In FIG. 1 ist die Abdeckhülse 4 in ihre proximale Endposition geschoben dargestellt, so dass die gesamte Ballonlänge zur Dilatation genutzt werden kann. Bei der in FIG. 2 dargestellten Vorrichtung zum Setzen eines ballonexpandierbaren Stents 9 ist die Abdeckhülse 4 zur Verkürzung der voll expandierbaren Ballonlänge teilweise über den deflatierten Dilatationsballon 3 geschoben worden, um die Ballonlänge an die Länge des Stents 9 anzupassen. Zur Einstellung einer besonders kurzen Ballonlänge kann die Abdeckhülse 4, wie in FIG. 3 gezeigt, in die Nähe ihrer distalen Endposition geschoben werden. Durch entsprechendes Verschieben der Abdeckhülse 4 können je nach praktischem Erfordernis auch andere, nicht in den Figuren dargestellte Ballonlängen erreicht werden.

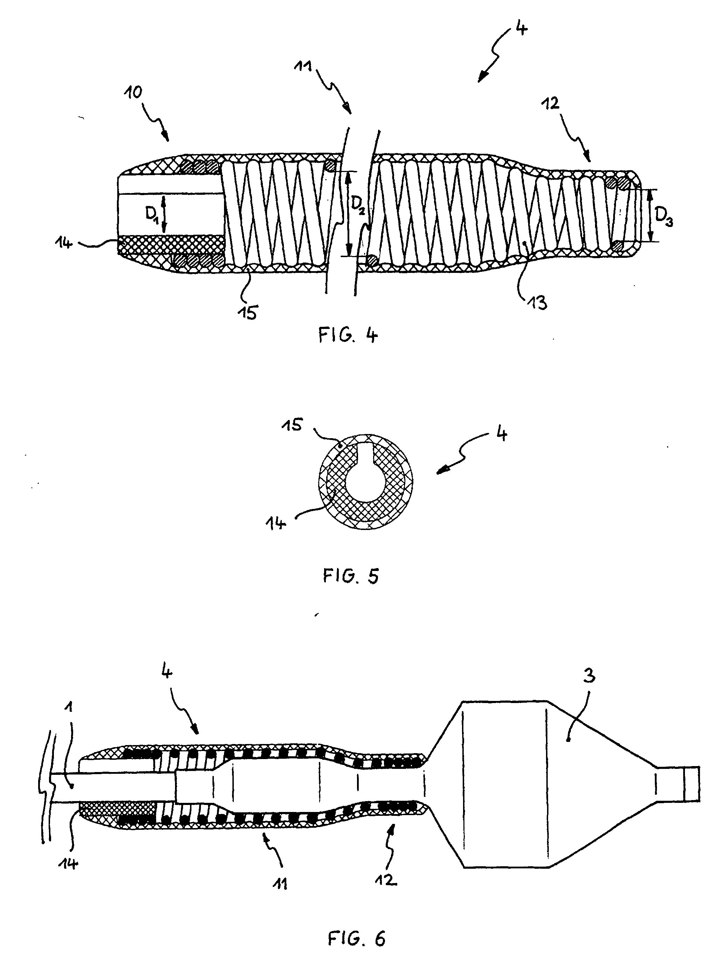

Die Abdeckhülse 4 weist gemäss FIG. 4 proximal einen ersten Abschnitt 10 mit einem ersten Innendurchmesser D₁ und einen sich daran anschliessenden zweiten Abschnitt 11 mit einem zweiten Innendurchmesser D₂ auf, der grösser als der erste Innendurchmesser D₁ ist. Distal vom zweiten Abschnitt 11 weist die Abdeckhülse 4 einen durchmesserstabilen dritten Abschnitt 12 mit einem dritten Innendurchmesser D₃ auf, der zwischen dem ersten Innendurchmesser D₁ und dem zweiten Innendurchmesser D₂ liegt. Der Übersichtlichkeit halber ist die Abdeckhülse 4 nicht in voller Länge dargestellt; bei einer Gesamtlänge der Abdeckhülse 4 von 20mm ist der dritte Abschnitt 12 nur etwa 1mm lang. Der zweite Abschnitt 11 und der dritte Abschnitt 12 sind einstückig mit einer aus einem schraubenlinienförmig gewickelten Drahtmaterial bestehende, durchmesserstabile Armierung 13 ausgebildet, um dem Dilatationsdruck des abgedeckten Ballonabschnitts standhalten zu können. Der erste Abschnitt 10 ist als Spannelement 14 ausgebildet und in den zweiten Abschnitt 11 eingesetzt. Stattdessen können auch alle Abschnitte 10, 11 und 12 der Abdeckhülse 4 einstückig ausgebildet sein, wobei die Armierung beispielsweise eine durchgehende Drahtspirale sein kann. Die Armierung 13 und das Spannelement 14 sind von einem Hüllschlauch 15 überzogen, damit die Abdeckhülse 4 eine glatte, atraumatische äussere Oberfläche aufweist. Die Struktur der inneren Oberfläche des Hüllschlauchs 15 stellt eine Einbettung für die Armierung 13 dar, während das Spannelement 14 distal von Windungen der Armierung 13 umspannt wird und proximal unmittelbar in den Hüllschlauch 15 eingeklebt ist. FIG. 5 zeigt einen Querschnitt durch die Abdeckhülse 4 im Bereich dieser Klebestelle. Das Spannelement 14 ist als längsgeschlitzte Spannhülse ausgeführt und vom Hüllschlauch 15 eingeschlossen. Der Schlitz dient zum Ausgleich von Durchmessertoleranzen des Katheterschaftes 1. Bei ausreichender Masshaltigkeit des Katheterschaftes 1 kann auf diesen Schlitz in der Spannhülse verzichtet werden.

Nach FIG. 6 umfasst das Spannelement 14 den Katheterschaft 1 proximal vom Dilatationsballon 3 so eng, dass ein genügend hoher Reibschluss zwischen Spannhülse und Katheterschaft 1 für eine sichere axiale Positionierung der Abdeckhülse 4 besteht. Der zweite Abschnitt 11 und der dritte Abschnitt 12 sind derart über den Dilatationsballons 3 geschoben, dass nach Inflatieren des Dilatationsballons 3 der durchmesserstabile dritte Abschnitt 12 proximal von einem teilexpandierten, abgedeckten Abschnitt und distal vom vollexpandierten, freien Abschnitt des Dilatationsballons 3 eingeschlossen ist. Dies sichert eine kontinuierlich einstellbare Ballonlänge, die sich nach einer gewählten Voreinstellung während des Einsatzes des Ballonkatheters nicht ändert.

### Bezugszeichenliste

- 1: Katheterschaft
- 2: distales Ende
- 3: Dilatationsballon
- 4: Abdeckhülse
- 6: Schulter
- 8: proximale Befestigungsstelle
- 9: Stent
- 10: erster Abschnitt
- 11: zweiter Abschnitt
- 12: dritter Abschnitt
- 13: Armierung
- 14: Spannelement
- 15: Hüllschlauch
- D₁: erster Innendurchmesser
- D₂: zweiter Innendurchmesser
- D₃: dritter Innendurchmesser

## Patentansprüche

1. Ballonkatheter mit einem rohrförmigen Katheterschaft (1), der ein proximales und ein distales Ende (2) aufweist, einem länglichen Dilatationsballon (3), der am Katheterschaft (1) in der Nähe des distalen Endes (2) angeordnet ist, und einer längs des Katheterschaftes (1) verschiebbaren, durchmesserstabilen Abdeckhülse (4), die einen den Katheterschaft (1) umfassenden ersten Abschnitt (10) mit einem ersten Innendurchmesser (D₁) und einen über den deflatierten Dilatationsballon (3) schiebbaren zweiten Abschnitt (11) mit einem zweiten Innendurchmesser (D₂) aufweist, der grösser als der erste Innendurchmesser (D₁) ist, **dadurch gekennzeichnet, dass** die Abdeckhülse (4) am distalen Ende des zweiten Abschnitts (11) einen durchmesserstabilen dritten Abschnitt (12) mit einem dritten Innendurchmesser (D₃) aufweist, der zwischen dem ersten Innendurchmesser (D₁) und dem zweiten Innendurchmesser (D₂) liegt.

2. Ballonkatheter nach Anspruch 1,
**dadurch gekennzeichnet, dass** die Abdeckhülse (4) von proximal auf den deflatierten Dilatationsballon (3) schiebbar ist, wobei der erste Abschnitt (10) proximal und der dritte Abschnitt (12) distal vom zweiten Abschnitt (11) der Abdeckhülse (4) angeordnet ist.

3. Ballonkatheter nach Anspruch 1 oder 2,
**dadurch gekennzeichnet, dass** der zweite Abschnitt (11) der Abdeckhülse (4) und einer der anderen Abschnitte (10,12) einstückig ausgebildet sind und der andere übsige Abschnitt (12,10) in den zweiten Abschnitt (11) eingesetzt ist.

4. Ballonkatheter nach Anspruch 3,
**dadurch gekennzeichnet, dass** einstückig ausgebildete Abschnitte der Abdeckhülse (4) eine durchmesserstabile, flexible Armierung (13) aufweisen.

5. Ballonkatheter nach einem der Ansprüche 3 oder 4,
**dadurch gekennzeichnet, dass** der eingesetzte Abschnitt (10,12) der Abdeckhülse (4) durch ein den Katheterschaft (1) eng umfassendes Spannelement (14) gebildet wird.

6. Ballonkatheter nach Anspruch 5,
**dadurch gekennzeichnet, dass** das Spannelement (14) eine längsgeschlitzte Spannhülse ist.

7. Ballonkatheter nach einem der Ansprüche 4 bis 6,
**dadurch gekennzeichnet, dass** die Abdeckhülse (4) einen die Armierung (13) und das Spannelement (14) überziehenden Hüllschlauch (15) aufweist.

8. Ballonkatheter nach Anspruch 2 oder nach einem der Ansprüche 3 bis 7, soweit diese auf Anspruch 2 zurückbezogen sind,
**dadurch gekennzeichnet, dass** der Katheterschaft (1) ein proximales Begrenzungselement und ein distales Begrenzungselement aufweist, zwischen welchen die Abdeckhülse (4) längs des Katheterschaftes (1) verschiebbar ist.

9. Ballonkatheter nach Anspruch 8,
**dadurch gekennzeichnet, dass** das proximale Begrenzungselement eine Schulter (6) am Katheterschaft (1) ist.

10. Ballonkatheter nach Anspruch 8 oder 9,
**dadurch gekennzeichnet, dass** das distale Begrenzungselement durch die proximale Befestigungsstelle (8) des Dilatationsballons (3) am Katheterschaft (1) gebildet wird.

11. Ballonkatheter nach Anspruch 9 oder 10,
**dadurch gekennzeichnet, dass** das distale Begrenzungselement durch den gefalteten, deflatierten Dilatationsballon (3) gebildet wird.

12. Ballonkatheter nach einem der Ansprüche 4 bis 11,
**dadurch gekennzeichnet, dass** die Armierung (13) aus einem schraubenlinienförmig gewickelten Drahtmaterial besteht.

13. Ballonkatheter nach einem der Ansprüche 4 bis 11,
**dadurch gekennzeichnet, dass** die Armierung (13) als rohrförmiges Drahtgeflecht ausgebildet ist.

14. Ballonkatheter nach einem der Ansprüche 4 bis 13,
**dadurch gekennzeichnet, dass** die Armierung (13) aus einem röntgendichten Material besteht.

15. Vorrichtung zum Setzen eines ballonexpandierbaren Stents (9), die einen Ballonkatheter nach einem der Ansprüche 1 bis 14 aufweist.

## Claims

1. Balloon catheter comprising a tubular catheter shaft (1) having a proximal and a distal end (2), an elongated dilation balloon (3) being arranged on the catheter shaft (1) in the vicinity of the distal end (2) thereof, and an indistensible covering sleeve (4) being movable along the catheter shaft (1), said covering sleeve having a first portion (10) with a first inner diameter (D₁) surrounding the catheter shaft (1) and a second portion (11) with a second inner diameter (D₂) being pushable over the deflated dilation balloon (3), said second inner diameter being larger than said first inner diameter (D₁), **characterized in that** the covering sleeve (4) comprises an indistensible third portion (12) with a third inner diameter (D₃) at the distal end of the second portion (11), said third inner diameter being between said first inner diameter (D₁) and said second inner diameter (D₂).

2. Balloon catheter according to claim 1, **characterized in that** the covering sleeve (4) is pushable from the proximal end onto the deflated dilation balloon (3), the first portion (10) being arranged proximally and the third portion (12) being arranged distally of the second portion (11) of the covering sleeve (4).

3. Balloon catheter according to claim 1 or 2, **characterized in that** the second portion (11) of the covering sleeve (4) and one of the other portions (10,12) are formed integrally as a single piece and the other remaining portion (12,10) is inserted in the second portion (11).

4. Balloon catheter according to claim 3,
**characterized in that** the portions of the covering sleeve (4) formed integrally as a single piece comprise an indistensible flexible armature (13).

5. Balloon catheter according to one of claims 3 or 4, **characterized in that** the inserted portion (10,12) of the covering sleeve (4) is formed by a tension element (14) closely surrounding the catheter shaft (1).

6. Balloon catheter according to claim 5, **characterized in that** the tension element (14) is a longitudinally slotted tensioning husk.

7. Balloon catheter according to one of claims 4 to 6, **characterized in that** the covering sleeve (4) comprises a jacket (15) covering the armature (13) and the tension element (14).

8. Balloon catheter according to claim 2 or according to one of claim 3 to 7 when dependent on claim 2, **characterized in that** the catheter shaft (1) comprises a proximal stop and a distal stop, between which the covering sleeve (4) is movable along the catheter shaft (1).

9. Balloon catheter according to claim 8, **characterized in that** the proximal stop is a shoulder (6) on the catheter shaft (1).

10. Balloon catheter according to claim 8 or 9, **characterized in that** the distal stop is formed by the proximal fixture (8) of the dilation balloon (3) on the catheter shaft (1).

11. Balloon catheter according to claim 9 or 10, **characterized in that** the distal stop is formed by the folded deflated dilation balloon (3).

12. Balloon catheter according to one of claims 4 to 11, **characterized in that** the armature (13) is made of a helically wound wire material.

13. Balloon catheter according to one of claims 4 to 11, **characterized in that** the armature (13) is formed by a tubular wire braiding.

14. Balloon catheter according to any one of claims 4 to 13, **characterized in that** the armature (13) is made of a radiopaque material.

15. Delivery device for a balloon expandable stent (9) comprising a balloon catheter according to one of claims 1 to 14.

## Revendications

1. Cathéter à ballon avec une tige de cathéter (1) de forme tubulaire qui présente une extrémité proximale et une extrémité distale (2), un ballon de dilatation (3) allongé qui est disposé sur la tige du cathéter (1) à proximité de l'extrémité distale (2) et un manchon de recouvrement (4) de diamètre stable, qui peut coulisser le long de la tige (1) du cathéter et qui présente une première partie (10) qui entoure la tige du cathéter (1) et avec un premier diamètre intérieur (D₁) et une deuxième partie (11) qui peut être passée au-dessus du ballon de dilatation (3) dégonflé, avec un deuxième diamètre intérieur (D₂) qui est plus grand que le premier diamètre intérieur (D₁), **caractérisé en ce que** le manchon de recouvrement (4) présente à l'extrémité distale de la deuxième partie (11) une troisième partie (12) de diamètre stable avec un troisième diamètre intérieur (D₃) qui est compris entre le premier diamètre intérieur (D₁) et le deuxième diamètre intérieur (D₂).

2. Cathéter à ballon selon la revendication 1, **caractérisé en ce que** le manchon de recouvrement (4) peut coulisser en position proximale sur le ballon de dilatation (3) dégonflé, la première partie (10) étant disposée du côté proximal et la troisième partie (12) du côté distal de la deuxième partie (11) du manchon de recouvrement (4).

3. Cathéter à ballon selon les revendications 1 ou 2, **caractérisé en ce que** la deuxième partie (11) du manchon de recouvrement (4) et l'une des autres parties (10, 12) sont réalisées d'un seul tenant, l'autre partie (12, 10) restante étant insérée dans la deuxième partie (11).

4. Cathéter à ballon selon la revendication 3, **caractérisé en ce que** les parties réalisées d'un seul tenant du manchon de recouvrement (4) présentent une armature (13) flexible de diamètre stable.

5. Cathéter à ballon selon l'une des revendications 3 ou 4, **caractérisé en ce que** la partie insérée (10, 12) du manchon de recouvrement (4) est formée par un élément de serrage (14) qui entoure étroitement la tige (1) du cathéter.

6. Cathéter à ballon selon la revendication 5, **caractérisé en ce que** l'élément de serrage (14) est un manchon de serrage fendu longitudinalement.

7. Cathéter à ballon selon l'une des revendications 4 à 6, **caractérisé en ce que** le manchon de recouvrement (4) présente une enveloppe souple (15) qui recouvre l'armature (13) et l'élément de serrage (14).

8. Cathéter à ballon selon la revendication 2 ou selon l'une des revendications 3 à 7, dans la mesure où celles-ci dépendent de la revendication 2, **caractérisé en ce que** la tige (1) du cathéter présente un élément proximal d'arrêt et un élément distal d'arrêt entre lesquels le manchon de recouvrement (4) peut coulisser le long de la tige (1) du cathéter.

9. Cathéter à ballon selon la revendication 8, **caractérisé en ce que** l'élément proximal d'arrêt est un épaulement (6) ménagé sur la tige (1) du cathéter.

10. Cathéter à ballon selon la revendication 8 ou 9, **caractérisé en ce que** l'élément distal d'arrêt est formé par un emplacement proximal de fixation (8) du ballon de dilatation (3) sur la tige (1) du cathéter.

11. Cathéter à ballon selon les revendications 9 ou 10, **caractérisé en ce que** l'élément distal d'arrêt est formé par un ballon de dilatation (3) replié et dégonflé.

12. Cathéter à ballon selon l'une des revendications 4 à 11, **caractérisé en ce que** l'armature (13) est formée d'un matériau en fil enroulé en spirale.

13. Cathéter à ballon selon l'une des revendications 4 à 11, **caractérisé en ce que** l'armature (13) est configurée comme treillis tubulaire de fil.

14. Cathéter à ballon selon l'une des revendications 4 à 13, **caractérisé en ce que** l'armature (13) est constituée d'un matériau opaque aux rayons X.

15. Dispositif de placement d'une endoprothèse (9) expansible par ballon qui présente un cathéter à ballon selon l'une des revendications 1 à 14.
